# EUROPEAN PATENT SPECIFICATION

(11) **EP 2 505 636 B1**
(45) Date of publication and mention of the grant of the patent: **04.03.2015**
(21) Application number: 11160790.9
(22) Date of filing: 01.04.2011
(51) Int. Cl.: C12N 1/06, C12N 1/12, C12P 7/64, C12M 1/00, C12P 1/00, C12R 1/89

(54) **A method for recovering a specific cellular component from a microorganism**
Verfahren zur Rückgewinnung eines spezifischen Zellkomponenten aus einem Mikroorganismus
Procédé pour la récupération de composants cellulaires spécifiques à partir d'un micro-organisme

(43) Date of publication of application: 03.10.2012
(73) Proprietor: Neste Oil Oyj, 02150 Espoo (FI)
(72) Inventor: Legrand, Catherine, 392 52 Kalmar (SE); Olofsson, Martin, 392 35 Kalmar (SE)
(74) Representative: Zacco Denmark A/S

(56) References cited:
- US-A1- 2011 076 748
- WU J T ET AL: "Cytotoxic effects of free fatty acids on phytoplankton algae and cyanobacteria", AQUATIC TOXICOLOGY, ELSEVIER SCIENCE PUBLISHERS BV, XX, vol. 80, no. 4, 30 December 2006 (2006-12-30), pages 338-345, XP025032270, ISSN: 0166-445X, DOI: 10.1016/J.AQUATOX.2006.09.011 [retrieved on 2006-12-30]
- YASUHIRO YAMASAKI ET AL: "Purification and characterization of a novel high molecular weight exotoxin produced by red tide phytoplankton, Alexandrium tamarense", JOURNAL OF BIOCHEMICAL AND MOLECULAR TOXICOLOGY, vol. 22, no. 6, 1 November 2008 (2008-11-01), pages 405-415, XP55006237, ISSN: 1095-6670, DOI: 10.1002/jbt.20253
- FRANCISCO A MACÍAS ET AL: "Allelopathic agents from aquatic ecosystems: potential biopesticides models", PHYTOCHEMISTRY REVIEWS, KLUWER ACADEMIC PUBLISHERS, DO, vol. 7, no. 1, 4 April 2007 (2007-04-04), pages 155-178, XP019556040, ISSN: 1572-980X, DOI: 10.1007/S11101-007-9065-1

## Description

### Field of the invention

The present invention relates to a method for recovering a specific cellular component from a microorganism, in particular for recovering lipids from a lipid-producing mircoorganism.

Also disclosed herein is an integrated system for rupturing lipid-producing algae cells.

### Background

Microorganisms such as algae, bacteria and fungi may contain triglycerides up to 80 % of their dry matter content. However, recovering lipids from biomass of mi-croorganisms with conventional methods can encounter unexpected problems in regard to residual biomass since the used cell-breaking-method for microorganisms may affect negatively to the subsequent use of residual biomass in other applications.

For example some autotrophic algae are lipid-rich, robust and easy to cultivate. The difficulty with some algal species relates to their cell wall, which is practically impossible to break efficiently with conventional methods for releasing the lipids of the cells while at the same time keeping the quality of the residual algal biomass high enough for continued processing and utilization.

As a consequence of presently used conventional methods, based on chemical solvents and often high temperature or pressure, the residual microorganism biomass such as algal cells cannot be used to many high-value applications, e.g. functional proteins (denaturation), food or feed (solvent residuals), which is important in order to maximise the value chain of algae and thereby to decrease price of the raw bio-oil. In addition, costs related to energy consumption (high temperature and pressure) and regeneration of large amount of solvents could be partly avoided.

WU J T et al., (2006) "Cytotoxic effects of free fatty acids on phytoplankton algae and cyanobacteria", AQUATIC TOXICOLOGY, vol. 80, no. 4, pages 338-345, discloses that potassium and phycobilins (cellular components) are released by the cyanobacteria upon treatment by algal toxin. Not all cellular compounds are released as chlorophyll is not detected in the medium. There is no disclosure of the release of lipids from cyanobacteria.

US 2011/076748 A1 (SALVO ROBERTO DI [US] ET AL) 31 March 2011 discloses a method of isolating cellular components (such as proteins and lipids) from algae by lysing cells in an ionic solution.

### Summary

The present invention tackles the above mentioned problems in a novel way by providing an alternative method for oil recovery from microbial biomass, especially from algae cells, based on solvent extraction, which is one of the major challenges in the applications using algal oil as feedstock for NExBTL (renewable diesel). Rupturing algae cells by means of unconventional biological means makes it possible to easily break of hard or otherwise unbreakable cell walls of some microorganisms. This method can potentially be applied also with other algal cells and microbial cells. The biochemical means used in the above mentioned novel biochemical method will preferably break cell walls / cell membranes of lipid-producing algae or other lipid-producing microbial species so, that biochemical means will not themselves harm environment but decompose when ended into nature.

The first aspect of the present method relates to a method for recovering lipids from a lipid-producing microorganism according to claim 1.

Also disclosed herein is an integrated system for rupturing lipids from a lipid-producing algae cells.

### Description

The present invention provides new techniques for rupturing cells of lipid-producing microorganisms especially algae and subsequent collection of secreted intracellular lipids from the water phase.

One of the main ideas behind the present invention is to use the biochemical rupturing capacity of highly cytotoxic algae on cell membrane or cell wall of lipid rich algae or other microorganisms and thereafter to collect lipid(s) from the mentioned lipid rich algae or other microorganisms by extracting oil droplets from water phase of algal or microbial aqueous slurry containing algal cell debris and released intracellular components.

Based on this main discovery the first aspect of the invention relates to a method for recovering lipids from a lipid-producing microorganism comprising the following steps:
- providing a biomass of a lipid-producing microorganism,
- rupturing the cell wall and / or cell membrane of said microorganism by algal cytotoxin
- thereby releasing lipids from the microorganism cell, and
- recovering said lipids .

Advantageously the above described method is used for recovering lipids from a lipid-producing algae. In this case the method comprises the following steps:
- providing a biomass of a lipid-producing algae,
- rupturing the cell wall and / or cell membrane of said algae by algal cytotoxin
- thereby releasing lipids from the algae cell, and
- recovering the lipids by extraction.

In an advantageous embodiment the cytotoxin is capable of rupturing the cell wall and / or cell membrane of the microorganism and also the cell wall and/ or cell membrane of at least one of the microalgae consisting of the species of the genera *Cryptomonas, Cyclotella, Dunaliella, Haematococcus, Phaeodactylum, Thalassiosira* and *Rhodomonas.*

By using algal cytotoxins in the above mentioned method, cell walls or cell membranes of microorganisms can be ruptured so that it is possible to recover cellular products without causing damage to residual microbial biomass, which can subsequently be used in other applications.

With this kind of gentle, biological cell wall/ cell membrane rupturing method, can also costs related to energy consumption or regeneration of solvents be decreased.

The method described above is especially suitable for rupturing cell walls of autotrophic algae belonging to different groups with semi-rigid or rigid wall (e.g. diatoms and certain green algae).

Also disclosed herein is an integrated system for rupturing lipids from lipid-producing algae cells, which comprises a growth vessel for lipid-producing algae and a growth vessel for a cytotoxin producing algae

Advantageously the integrated system for rupturing lipid-producing algae cells comprises a growth vessel for lipid-producing algae and a growth vessel for a cytotoxin (s) producing algae, which are capable of rupturing the cell wall and/ or cell membrane of at least one of the microalgae consisting of the species of the genera *Cryptomonas, Cyclotella, Dunaliella, Haematococcus, Phaeodactylum, Thalassiosira* and *Rhodomonas.*

In said integrated system lipid-producing algae is cultivated under conditions suitable for producing lipids and said cytotoxin-producing algae is cultivated under conditions suitable for producing cytotoxins. Produced cytotoxins will be added to the growth vessel containing lipid-producing algae, in sufficient amount to rupture cell walls and/or cell membranes of mentioned lipid-producing algae thereby releasing the lipid component from the cells and recovering lipids from the other cell components.

In an alternative embodiment of the above integrated system, lipid-producing algae and cytotoxin-producing algae are both cultivated in the same growth vessel and in such an environment that the cytotoxin production is suppressed. After the biomass of cytotoxin-producing algae is at a suitable level, the production of exotoxins is triggered for example by using suitable stress conditions.

In this specification is described also a composition used for rupturing lipid-producing algae cells, which comprises an algal cytotoxin , which is selected so, that it is capable of rupturing the cell wall and /or cell membrane of the lipid-producing algae.

Advantageously algal cytotoxin (s) is selected so, that it is capable of rupturing the cell wall and / or cell membrane of at least one of the following microalgae selected from the group consisting of *Cryptomonas* spp.; *Cyclotella* spp.; *Dunaliella* spp.; *Haematococcus* spp.; *Phaeodactylum* spp.; *Thalassiosira* spp. and *Rhodomonas* spp. thereby releasing the lipid component from said lipid-producing algae cells.

As used herein, the definition "algal cytotoxin" refers to a toxic algal substance originating from algae, which toxic algal substance is able to rupture cell wall(s) and /or cell membrane(s) of algae or other microorganisms. These algal cytotoxins include for example extracellular algal toxins (exotoxins), excreted by microalgae. Herein the term "cytotoxin" is used to specify the cell wall or/and cell membrane rupturing action of an algal toxins released into the medium surrounding algal cells since algal toxins may also cause the death of microorganisms with other mechanisms without actually rupturing the cell walls/cell membranes of said microorganisms.

Toxic algal substances, as used herein, means same as algal toxins.

The chemical structure of algal cytotoxins differ considerably since each algae may produce several different toxic algal substances. Therefore the mechanism by which each algal toxic substance acts on the algae and other microorganisms differ considerably. In case an algal toxic substance kills microorganisms, one common killing mechanism is the lysis of cell wall / cell membrane. The algal cytotoxins can usually be found among the algae, which excrete, in some conditions, extracellular toxins into medium surrounding them.

One of these main sources is marine or freshwater plankton which comprises algae groups including diatoms, cyanobacteria, dinoflagellates, prymnesiophytes and raphidophytes, and which can produce potentially cytotoxic algal toxins into their surroundings. Whether all algal exotoxins really have the cytotoxic effect against certain microorganisms should be tested separately in regard to test probe that consists of microorganism of the interest and also at least one of the microalgae, bacteria or yeast mentioned in this description. Advantageously mentioned test probe includes microalgae selected from the group consisting of the genera *Cryptomonas, Cyclotella, Dunaliella, Haematococcus, Phaeodactylum, Rhodomonas and Thalassiosira.* If an exotoxin is cytotoxic against tested microorganisms it will rupture their cell walls/cell membranes and also cell wall/cell membrane of at least one microalgae listed in this description.

The algal cytotoxins, which can rupture or lyse the cell wall/cell membrane of microorganisms, can be derived preferably from algae species belonging to above mentioned groups: diatoms, cyanobacteria, dinoflagellates, prymnesiophytes or raphidophytes.

In the following are given some examples of algal species belonging to the above mentioned algal genera whose algal toxins or toxic secondary metabolites may be cytotoxic, that is, they may be able to rupture cell membranes/cell walls of at least algae (including cyanobacteria), bacteria or fungi including also cell wall and/or cell membrane of at least one of the following microalgae selected from the group consisting of genera *Cryptomonas, Cyclotella, Dunaliella, Haematococcus, Phaeodactylum, Rhodomonas, and Thalassiosira.*

Potentially cytotoxic algae, which belong to cyanobacteria include the genera *Anabaena, Aphanizomenon, Calothrix, Cylindrospermopsis, Fisherella, Gomphosphaeria, Hapalosiphon, Microcystis, Nodularia,* and *Nostoc.* Examples of potentially cytotoxic algae belonging to dinoflagellates are *Alexandrium, Coolia, Dinophysis, Heterocapsa, Karlodinium, Karenia, Ostreopsis, Peridinium and Prorocentrum.* Examples of potentially cytotoxic algae belonging to prymnesiophytes are *Chrysochromulina, Phaeocystis and Prymnesium.* Examples of potentially cytotoxic diatoms are *Pseudonitzschia* and *Nitzschia* and examples of potentially cytotoxic raphidophytes are *Heterosigma and Chattonella.*

From above mentioned algae species, *Alexandrium* produces stabile toxic algal substances, which can be used as cytotoxins since they are able to rupture and lyses a whole range of algal cells, including for example those, belonging to flagellates and diatoms. The most preferable toxic algal substances for use as algal cytotoxins, are those whose toxicity disappears fast from the water phase by chemical effect such as temperature or light, or biological effect such as bacterial degradation. These kind of algal cytotoxins are for example cytotoxins excreted by the genus *Prymnesium,* which is a fast-growing haptophyte. For example the algal cytotoxins produced by the mixotrophic *P. parvum* may be degraded by the effect of sunlight and UV-radiation.

In addition to algae, many algal toxins or toxic secondary metabolites of algae have also cytotoxic effect on cell membranes/cell walls of fungi or bacteria such as heterotrophic bacteria (Phycologia (2003) Vol 42 (4) 406 -419). If these fungi or bacteria can accumulate high intracellular lipid content, these lipids may be worth of recovering by using the method and means according to present invention.

As used herein the definition "rupturing" cells of algae or other microorganisms refers to a process which damages the cell walls or cell membranes of algae or other microorganisms by means of the action of an algal cytotoxin and which results to destruction, lysis, degradation, decomposition or loss of integrity of the cell walls/ cell membranes in such an extent that it allows releasing of oil / lipids from the interior of mentioned algal or microbial cells.

As used herein the term "lipid" refers to a fatty substance, whose molecule generally contains, as a part, an aliphatic hydrocarbon chain, which dissolves in nonpolar organic solvents but is poorly soluble in water.

Lipids are an essential group of large molecules in living cells. Lipids comprise, for example, fats, oils, waxes, wax esters, sterols, terpenoids, isoprenoids, carotenoids, polyhydroxyalkanoates, fatty acids, fatty alcohols, fatty acid esters, phospholipids, glycolipids, sphingolipids and acylglycerols, such as monoglycerides (monoacylglycerol), diglycerides (diacylglycerol) or triglycerides (triacylglycerol, TAG). In the present invention desired lipids to be recovered in the product include fats, oils, waxes and fatty acids and their derivatives.

As used here the term "biomass" is meant biomass derived from a culture containing microorganisms including bacteria, cyanobacteria, fungi such as yeasts, filamentous fungi and moulds, archaea, protists; microscopic plants such as algae, microalgae or plankton. This term includes also a ready-made, frozen or otherwise previously worked biomass, which is subsequently used in this method.

Definition "providing a biomass" comprises herein the use of a biomass derived from a culture of microorganisms or the use of a ready-made frozen or otherwise previously worked biomass.

Most lipid-producing microorganisms are unicellular i.e. single-celled, however, some microscopic multicellular organisms are also able to accumulate lipids. The microorganisms readily accumulate lipids or have been genetically modified to accumulate lipids or to improve accumulation of lipids. In a preferred embodiment of the present invention lipid containing microbial biomass is selected from the group of bacteria, cyanobacteria, fungi such as yeasts, filamentous fungi, moulds, archaea, protists; microalgae, bacteria, fungi, filamentous fungi, moulds and yeasts.

Preferably, suitable microalgae comprise one or more representatives from the following taxonomic classes: *Chlorophyceae*, *Cryptophyceae* (recoiling algae), *Chrysophyceae, Diatomophyceae* (diatoms), *Dinophyceae* (dinoflagellates), *Euglenophyceae, Eustigmatophyceae, Pavlovophyceae, Pedinophyceae, Prasinophyceae, Prymnesiophyceae* (haptophyte algae) and *Raphidophyceae.*

In a more preferred embodiment the microbial biomass comprises freshwater and marine microalgae genera comprising *Achnantes, Agmenellum, Amphiprora, Amphora, Anabaena, Anabaenopsis, Ankistrodesmus, Arthrospira, Attheya, Boeklovia, Botryococcus, Biddulphia, Brachiomonas, Bracteococcus, Carteria, Chaetoceros, Characium, Chlamydomonas, Cricophaera, Crypthecodinium, Cryptomonas, Chlorella, Chlorococcum, Chrysophaera, Coccochloris, Cocconeis, Cyclotella,Cylindrotheca, Dermocarpa, Dunaliella, Ellipsoidon, Entomoneis, Euglena, Eremosphaera, Extubocellulus, Franceia, Fragilaria, Gleocapsa, Gleothamnion, Hantzschia, Haematococcus, Hormotilopsis, Hymenomonas, Isochrysis, Lepocinclis, Melosira, Minidiscus, Micractinum, Microcystis, Monallanthus, Monoraphidium, Muriellopsis, Nannochloris, Nannochloropsis, Navicula, Neochloris, Nephroselmis, Nitzschia, Nodularia, Nostoc, Ochromonas, Oedogonium, Oocystis, Oscillatoria, Papiliocellulus, Parachlorella, Pascheria, Pavlova, Peridinium, Phaeodactylum, Phagus, Plankthothrix, Platymonas, Plectonema, Pleurochrysis, Phormidium, Pleurosigma, Porphyridium, Prymnesium, Pseudochlorella, Pyramimonas, Pyrobotrus,Radiosphaera, Rhodomonas, Rhodosorus, Sarcinoid, Scenedesmus, Schizochytrium, Scrippsiella, Seminavis, Skeletonema, Spirogyra, Spirulina, Stichococcus, Synechococcus, Synechocystis, Synedra, Tetraedron, Tetraselmis, Thalassiosira, Trachyneis, Traustrochytrium, Trentepholia, Ulkenia, Viridiella, Volvox* and *Xenococcus.*

The method was found to be particularly effective with microalgae selected from the group consisting of *Chlorella* spp. such as *Chlorella pyrenoidosa; Dunaliella* spp. such as *Dunaliella tertiolecta, Dunaliella marina and Dunaliella salina; Nannochloropsis* spp. *and Phaeodactylum* spp. such as *Phaeodactylum tricornutum* capable of accumulating a high lipid content.

In another preferred embodiment the microbial biomass comprises fungal species, especially filamentous fungal species, belonging to the following genera *Aspergillus, Chaetomium, Claviceps, Cladosporidium, Cunninghamella, Emericella, Fusarium, Glomus, Humicola, Malbranchea Mortierella, Mucor, Paecilomyces, Penicillium, Pythium, Rhizopus, Trichoderma, Zygorhynchus and Ustilago* especially those species having high amounts of lipids and essential fatty acids. Preferably, microbial biomass comprises *Mortierella isabellina, Mucor, Aspergillus or Rhizopus.*

In yet another preferred embodiment the microbial biomass comprises oleaginous yeast belonging to the following genera *Candida* such as *Candida curvata, Clavispora, Cryptococcus, such as Cryptococcus curvatus, Deparyomyces, Endomycopsis, Galactomyces, Hansenula, Kluyveromyces, Lipomyces* such as *Lipomyces starkeyi, Pachysolen, Pichia,* such as *Pichia stipitis, Rhodosporidium,* such as *Rohodosporidium toruloides, Rhodotorula,* such as *Rhodotorula glutinis, Saccharomyces, Waltomyces, Yarrowia,* such as *Yarrowia lipolytica* and *Trichosporon* such as *Trichosporon cutaneum or Trichosporon pullulans* which readily accumulate lipids or have been genetically modified to produce lipids. Most preferably, yeasts comprise *Lipomyces, Rhodosporidium* or *Cryptococcus.*

In yet another preferred embodiment the microbial biomass comprises bacteria belonging to the following genera *Acinetobacter, Actinobacter, Alcanivorax, Aerogenes, Anabaena, Arthrobacter, Bacillus, Clostridium, Dietzia, Escherichia, Flexibacterium, Gordonia, Micrococcus, Mycobacterium, Nocardia, Nostoc, Oscillatoria, Pseudomonas, Rhodococcus, Rhodomicrobium, Rhodopseudomonas, Shewanella, Shigella, Streptomyces and Vibrio.* Most preferably bacteria comprise *Rhodococcus opacus, Acinetobacter, Nocardia or Streptomyces.*

By the term such as "advantageous", "preferred", "preferential" or "special" is meant herein that the selected subject matter is advantageously but not necessarily used in this connection because there is some advantages relating to its use. However there can be also some disadvantages involving to the use of subject matter that is "advantageous", "preferred", "preferential" or "special" and therefore these terms should not be interpreted restrictive in any way.

### General method

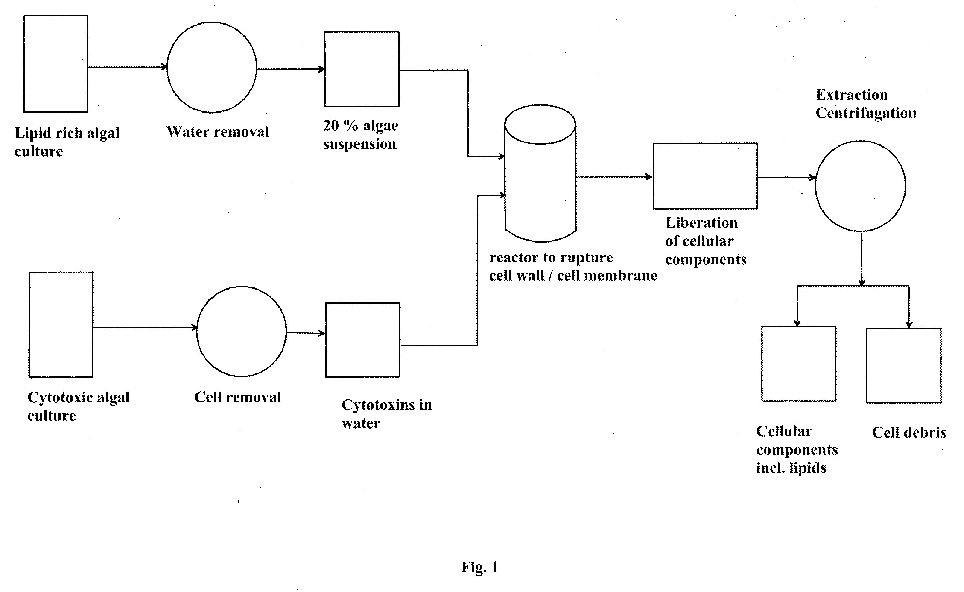

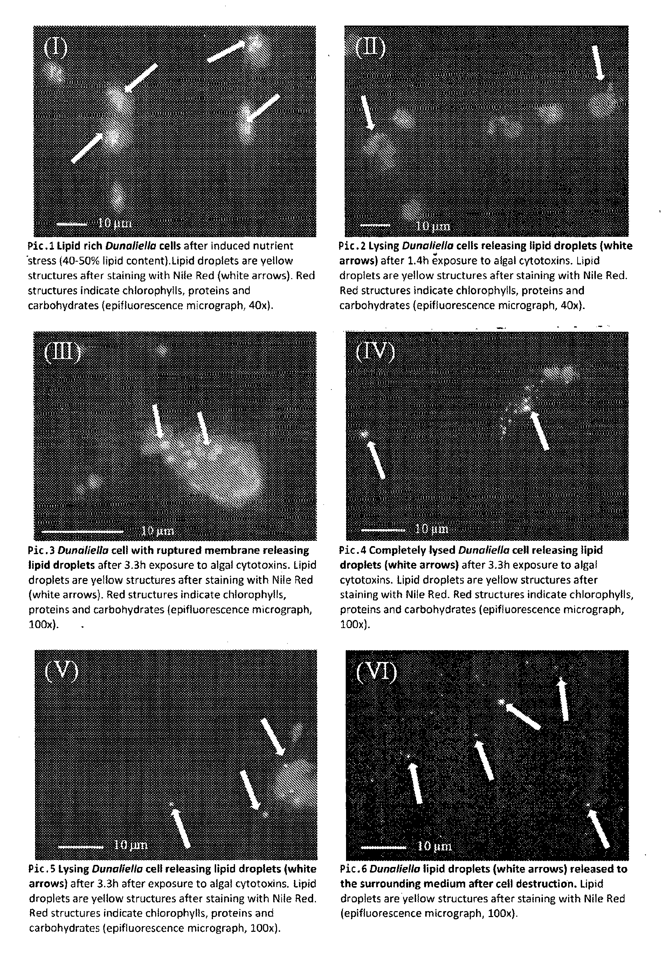

The general method used for recovering products such as lipids from microbial biomass, especially from algae cells is outlined in figure 1.

Although this method is described for recovering lipids from microalgae the same kind of method may be used also for recovering other products from suitable microbial biomasses as far as their cell walls and / or cell membranes are breakable or rupturable by toxic algal substances, which can also break or rupture at least one microalgae selected from the group consisting of the genera *Achnantes, Agmenellum, Amphiprora, Amphora, Anabaena, Anabaenopsis, Ankistrodesmus, Arthrospira, Attheya, Boeklovia, Botryococcus, Biddulphia, Brachiomonas, Bracteococcus, Carteria, Chaetoceros, Characium, Chlamydomonas, Cricophaera, Crypthecodinium, Cryptomonas, Chlorella, Chlorococcum, Chrysophaera, Coccochloris, Cocconeis, Cyclotella, Cylindrotheca, Dermocarpa, Dunaliella, Ellipsoidon, Entomoneis, Euglena, Eremosphaera, Extubocellulus, Franceia, Fragilaria, Gleocapsa, Gleothamnion, Hantzschia, Haematococcus, Hormotilopsis, Hymenomonas, Isochrysis, Lepocinclis, Melosira, Minidiscus, Micractinum, Microcystis, Monallanthus, Monoraphidium, Muriellopsis, Nannochloris, Nannochloropsis, Navicula, Neochloris, Nephroselmis, Nitzschia, Nodularia, Nostoc, Ochromonas, Oedogonium, Oocystis, Oscillatoria, Papiliocellulus, Parachlorella, Pascheria, Pavlova, Peridinium, Phaeodactylum, Phagus, Plankthothrix, Platymonas, Plectonema, Pleurochrysis, Phormidium, Pleurosigma, Porphyridium, Prymnesium, Pseudochlorella, Pyramimonas, Pyrobotrus,Radiosphaera, Rhodomonas, Rhodosorus, Sarcinoid, Scenedesmus, Schizochytrium, Scrippsiella, Seminavis, Skeletonema, Spirogyra, Spirulina, Stichococcus, Synechococcus, Synechocystis, Synedra, Tetraedron, Tetraselmis, Thalassiosira, Trachyneis, Traustrochytrium, Trentepholia, Ulkenia, Viridiella, Volvox* and *Xenococcus* or bacteria selected from the group consisting of the genera *Acinetobacter, Actinobacter, Aerogenes, Alcanivorax, Arthrobacter, Bacillus, Clostridium, Cupriviadus, Dietzia, Gordonia, Escherichia, Flexibacterium, Micrococcus, Mycobacterium, Nocardia, Pseudomonas, Ralstonia, Rhodococcus, Rhodomicrobium, Rhodopseudomonas, Shewanella, Shigella, Streptomyces, Wautersia* and *Vibrio*
or at least one yeast selected from the group consisting of the genera *Candida, Clavispora, Cryptococcus, Deparyomyces, Endomycopsis, Galactomyces, Hansenula, Kluyveromyces, Lipomyces, Pachysolen, Pichia, Rhodosporidium, Rhodotorula, Saccharomyces, Waltomyces, Yarrowia* and *Trichosporon,* or at least one filamentous fungi selected from the group consisting of the genera *Absidia, Aspergillus, Blakeslea, Chaetomium, Cladosporium, Claviceps, Cladosporidium, Cunninghamella, Emericella, Entomophthora, Fusarium, Gibberella, Glomus, Humicola, Malbranchea, Mucor, Mortierella, Paecilomyces, Penicillium, Puccia, Pythium, Rhizopus, Saprolegnia, Trichoderma, Thermomycs, Ustilago* and *Zygorhynchus.*

Advantageously these toxic algal substances will break or rupture microalgae selected from the group consisting of *Cryptomonas* spp.; Cyclotella spp.; *Dunaliella* spp.; *Haematococcus* spp.; *Phaeodactylum* spp.; *Thalassiosira* spp. and *Rhodomonas* spp.

Other suitable microbial biomasses have been given above when discussing the meaning of definition "biomass".

The method outlined in figure 1 can be used for recovering lipids from the same microorganisms (microalgae, bacteria, yeast or filamentous fungi) mentioned above, at least one of whose cell wall and / or cell membrane should be also breakable or rupturable by toxic algal substances. These microorganisms include also following microalgae belonging to genera: *Cryptomonas, Cyclotella, Dunaliella, Haematococcus, Phaeodactylum, Thalassiosira* spp. and *Rhodomonas* spp. , or following bacteria *Rhodococcus opacus, Acinetobacter, Nocardia* or *Streptomy*ces., or following yeasts *Cryptococcus curvatus, Rhodosporidium toruloides, Rhodotorula glutinis, Yarrowia lipolytica, Pichia stipitis, Candida curvata, Lipomyces starkeyi* or *Lipomyces lipofer* and *Trichosporon cutaneum* or *Trichosporon pullulans* and following filamentous fungi: *Absidia spinosa,* species belonging to the genus *Aspergillus* such as *A*. *ficheri, A. flavus, A. nidulans, A. ochraceus, A. oryzae* and *A*. *terrius, Blakeslea trispora, Chaetomium globosum, Cladosporidium herbarum, Claviceps purpurea,* species belonging to the genus *Cunninghamella* such as *C*. *echinulata, C. japonica* and *C*. *elegans, Entomophthora coronata, Fusarium bulbigenum, Fusarium graminearum, Fusarium sp., Gibberella fujikuroi, Glomus caledonius, Humicola lanuginosa,* species belonging to the genus *Mucor* such as *M*. *circinelloides, M. plumbeus* and *M. rouxii,* species belonging to the genus *Mortierella* such as *M*. *isabellina, M. alpina* and *M*. *ramanniana,* species belonging to the genus *Penicillium* such as *P. javanicum, P. lilacinum, P. spinulosum* and *P*. *soppii, Puccia coronata, Pythium ultimum, Rhizopus delemar, Rhizopus oryzae, Ustilago zeae, Zygorhynchus moelleri, Malbranchea pulchella, Myrothecium sp., Sclerotium bataticola, Pellicularia practicola, Sphacelothea reiliana, Tyloposporidium ehren bergii, Achyla americana, Lepista nuda, Tilletia controversa and Cronartium fusiform.*

Algae can grow either in light conditions or dark conditions. In light conditions algal growth is photoautotrophic or autotrophic and the light energy is required for growth. In dark conditions the algal growth is heterotrophic which means, that other kind of energy than light is required for growth. Lipid-producing algae can be cultivated in both of these conditions. However autotrophic growth and heterotrophic growth requires different growth conditions and nutrients, which should be taken into account when considering cultivation.

During the heterotrophic and autotrophic growth certain growth conditions and nutrients can be used for promoting oil production inside the algae cells. Additionally the oil production of some lipid-producing algae can be enhanced by cultivating lipid-producing algae under cultivation conditions comprising a stress induction phase. Possibilities for causing stress induction phase are for example, light deprivation, injection of reactive oxygen, pH or nutrition changes or chemical addition. For example by changing the proportion of nitrogen to phosphorus during the growth phase of algae can boost remarkable lipid production as demonstrated also in examples 1 - 6 below.

The microbial biomass to be processed may be obtained directly from cultivation or growth system such as a growth vessel. When method presented in figure 1 is used for recovering oil from lipid-producing algae, the method is usually commenced by separately culturing lipid-producing algae and a cytotoxin-producing algae in different growth vessels.

Alternatively the method presented in figure 1 can be modified so, that lipid-producing algae and toxin-producing algae are cultivated in a same growth vessel. However, the culturing conditions are such, that the extracellular toxin production is suppressed. After the biomass of toxin-producing algae is at a suitable level, the production of exotoxins is triggered by changing culturing conditions, for example by subjecting toxin-producing algae to suitable stress induction phase.

Growth vessel as used herein means a closed solar photoreactor or a closed, artificially illuminated photoreactor, an open container or raceway, or a reservoir or a natural or artificial water pond. Reservoir or water pond may contain natural fresh water or natural seawater or artificial seawater.

Each growth vessel may include a carbon dioxide source or carbon dioxide can be added into the growth vessel during the cultivation for adjusting pH. Growth environment can also contain suitable temperature controlling, aerating and circulating means. It may also be necessary to supply nutrients and modify nutrient content to each other. If algae are cultivated in a closed photoreactor, the reactor should be provided with illumination.

The lipid-producing algae to be cultured in a growth vessel may comprise of a single algal species or a mixed combination of two or more algal species.

Wide variety of algae can be used for production of biomass, from which lipid or oil can be recovered. The method is suitable for autotrophic, heterotrophic or mixotrophic algae. Most common lipid-producing algae can be found from the groups of diatoms, chlorophytes (green algae), cyanobacteria (blue-green algae), chrysophytes (golden-brown algae), dinoflagellates and haptophytes. Suitable lipid-producing algae can be found among those algae mentioned above or from discussion relating to the term "biomass".

Microbial biomass to be processed is treated by generally known methods, such as centrifugation, filtration, decanting, floatation or sedimentation possible assisted by flocculation and water evaporation to remove excess water or aqueous growth solution. Microalgae, bacteria, archaea, filamentous fungi, mould or yeast biomass is preferably filtered or centrifuged before processing. On the other hand, biomass from solid state cultivation, immobilized cultivation or the like may be used by slurring it into aqueous media, if necessary.

By the term "wet" is meant microbial biomass which originates from aqueous cultivation solution and from which excess water is removed by common low energy consuming water removal processes such as filtering or the like and which is not specifically dried. Alternatively, solid dry microbial biomass may be slurried into an aqueous form.

In the process described in figure 1, the lipid-producing algae are first cultivated in a first growth vessel and the cultivated algae cells are then partially dried by removing excess water by evaporation, sedimentation, centrifugation which are possibly assisted by flocculation.

The algal cytotoxins used in this method can be produced by a suitable algae mentioned above when discussing the definition "algal cytotoxin". Preferably algal cytotoxins are produced by a single phytoplankton species naturally present in marine or freshwater environment or by the mixture of two or more phytoplankton species.

The cytotoxin-producing algae are cultivated in a second growth vessel (that is, in a closed photoreactor, open container or a reservoir or a natural or artificial water pond). The growth conditions and nutrients should be adjusted according to requirements of algal species to be grown. Algal cytotoxins producing algae comprise different species with different requirements as to their growth conditions, nutrients and nutrient ratios. For example cytotoxin-producing dinoflagellates include species, ranging from obligate autotrophs to mixotrophs and therefore it exists a wide variety of factors, which affect their toxicity and growth. For example pH, temperature, salinity of growth medium and nutrient limitations can affect the toxicity of algal cytotoxins. After cytotoxin-producing algae have been cultivated in a growth vessel, the algal slurry is filtered for removing algal cells and recovering cell-free filtrate, which contains the algal cytotoxins.

The filtrate, which contains algal cytotoxins, is then added into partially dried algal biomass. In one embodiment the algal cytotoxins and water containing filtrate is added to the dried algal biomass containing algae cells in such an amount, that the proportion of algal cytotoxins producing algae cells, from which the algal toxins have been recovered, to lipid-producing algae cells will be from 1:100 000 to 1:10 preferably from 1:1000 to 1: 100 (cell /cell). This virtual cell-proportion depends on the quality and quantity of cytotoxins present in the filtrate and the toxicity of cytotoxins against lipid-producing algae cells. After cytotoxin(s) containing aqueous filtrate has been added into partially dried algae cells the solids content of this aqueous algal suspension is preferable about 20 wt-% the rest of suspension being mostly water.

Alternatively the cytotoxin-producing algal culture is added without filtration or extraction to partially dried lipid-rich algal cells. It is possible also to combine the lipid-producing algal culture to the cytotoxin-producing algal culture without first processing neither of these algal cultures and thereafter to recover the lipids from this combined algal suspension.

After algal cytotoxins have been added into the lipid-rich algal culture, cytotoxic substances will affect to the cell walls/ cell membranes of the algae cells by rupturing them. Usually the cell walls/cell membranes of algae lyse completely due to effect of cytotoxin(s) and lipids will be released into slurry of algal debris, which includes lipids, cell wall debris, intracellular products, enzymes, by-products etc.

The lipids can be removed from mentioned aqueous slurry of algal debris by conventional methods such as extraction, centrifugation and/or filtering, for example by means of an extraction column.

The algal debris (biomass), which has been left after removal of lipids, is in a good condition and can be utilized in subsequent reprocessing stages for producing other valuable products. The recovered lipids can be used in the production of biodiesel, renewable diesel, jet fuel, gasoline or base oil components.

The method can be used also for recovering oil / lipids from the microorganisms (microalgae, bacteria, yeast or filamentous fungi) mentioned above. Especially suitable this method is for recovering oil from following algal species: *Haematococcus* spp.; *Dunaliella* spp. such as *Dunaliella tertiolecta, D. marina, D. salina (green algae); Phaeodactylum* spp. such as *Phaeodactylum tricornutum* (diatom), *Thalassiosira* spp. such as *T. pseudonana, T. weissflogii* and *Cyclotella* spp. (diatom); *Cryptomonas* spp. and *Rhodomonas* spp. *such* as *Rhodomonas salina and Rhodomonas lacustris* (recoiling algae). These microalgae are capable of accumulating high lipid content.

### Examples

### Lipid-producing algae

Chlorophyte *Dunaliella tertiolecta* (CCMP 1320)

Diatom *Phaeodactylum tricornatum* (CCMP 2928)

### Cytotoxin-producing algae

Preferable the used cytotoxin is degradable in the environment by chemical or biological effect such as temperature, sunlight (or artificial UV-light), or bacteria, or binding to organic matrixes. However, for the purpose of illustrating the validity of our method in rupturing the cell walls of lipid-producing algae and subsequent lipid recovery from aqueous algal cell debris we used here more stable algal cytotoxins originating from the dinoflagellate genus *Alexandrium.* These cytotoxins were produced by *Alexandrium tamarense* during exponential growth in a photoreactor supplied with an artificial illumination. During the cultivation of lipid-producing algae was introduced a stress inducing phase by restricting the access to nutrients during late growth phase.

### Examples 1 and 2

### Culturing of lipid-producing algae and potentially cytotoxic algae

Two oil-producing algae, the chlorophyte *Dunaliella tertiolecta* (CCMP 1320) and the diatom *Phaeodactylum tricornatum* (CCMP 2928) were cultivated in f/2 medium (N: P = 24) prepared from filtered artificial seawater (35 ‰) in aerated batch cultures (2-10 L). Oil-producing algae were grown under controlled conditions at 20°C on a 16:8 h light-dark cycle a under cool white fluorescent light at an irradiance of 220 µE m-² s-¹. CO₂ was injected daily to lower the pH from 9-9.5 to 8-8.5, and grown until exponential phase at a biomass of 3 x 10⁶-5 x 10⁶ cells mL ⁻¹ containing 10-20 % lipids (dry w/dry w).

To boost lipid accumulation in the cells, a stress induction phase was commenced by inducing nutrient stress in the oil-producing algal cultures through harvesting 20-30 % of the culture volume before replenishing the culture with modified f/2 (N: P = 2). Lipid content reached 40-50 % cellular lipid (dry w/dry w) over a period of 1-2 days.

Quantification of lipid content was carried out using a modified Bligh & Dyer (1959) method.

Potentially cytotoxic algal substances producing *Alexandrium tamarense* was grown in K-medium (N: P = 24) prepared from filtered seawater (salinity 32) in 2 L batch cultures under controlled conditions at 15°C on a 16:8 h light-dark cycle under cool white fluorescent light at an irradiance of 65 µE m⁻² s⁻¹.

### Examples 3 and 4

### Lipid rich algae were harvested through centrifugation (20 min., 100 x g)

Algal toxic substances from A. *tamarense* (Tillmann & John, 2002) were released from the cells to the surrounding medium. To collect algal toxic substances, cell-free filtrate of *A*. *tamarense* was prepared from dense stationary phase cultures (8-20 x 10³ cells mL⁻¹) by gently filtering the culture through a 10 µm mesh nylon net.

### Example 5

### Partial drying of the algal cells

Lipid rich algal wet biomass was obtained after centrifugation (40 mL) of the target cultures and re-suspension (*Dunaliella*: 0.2-1.4 x 10⁸ cells mL or 4.5-5.8 g L ⁻ dry weight; *Phaeodactylum*: 2.2 -4.5 x 10⁸ cells mL⁻¹). The supernatant was discarded except for 3-4 ml that was retained in the centrifugation tube and used for re-suspension of the pelleted biomass. Re-suspension was carried out in order to obtain a practical suspension easy to work with.

### Example 6

### Cell membrane rupture

Lipid rich algae cell membrane was biologically ruptured through the addition of toxic algal substances originating to *A*. *tamarense* (cell-free filtrate from examples 3 and 4). A combination of dose response assays and time series were carried out to define the optimal parameters (dose: target ratio and exposure time) resulting in the rupture of the cell membrane of the target (*Dunaliella, Phaeodactylum*). Algal cytotoxins were added to target cells in the range of dose: target ratio (cell: cell) of 1:1000 to 1: 100, and incubated over 24 h with sampling every 15 minutes over 4h, and a final sampling at 24h. After the addition of algal cytotoxins to target cells, these cells were immediately stained with the fluorochrome Nile Red (3.9 µM final concentration) to stain the cellular lipid droplets (Nile Red: A selective fluorescent stain for intracellular lipid droplets, Greenspan et al., The Journal of Cell Biology, Vol 100, 965-973, March 1985). Stained cells were stored 10 min in the dark prior to observation of the cells in epifluorescence microscopy (blue light). The process of lipid release from the cellular matrix was followed using 15 min intervals over 4 hours. After short exposure (1-4 h) to algal cytotoxins, lipid-rich algae cell lysis occurs and cells break open thus confirming cytotoxic effect of mentioned toxic algal substances. Microscopic observation revealed that within 1 h of exposure to these algal cytotoxins, the donor cell membrane started to rupture in both *Dunaliella* and *Phaeodactylum.* After rupture of the cell membrane (3-4 h), oil droplets were released from the cell matrix into the surrounding water. Lipid release can be uneven in different cells; oil droplets could still be observed in the cellular matrix after 4 h. This process was observed using epifluorescence microscopy (40-100X, blue light at 488 nm). Oil droplets, stained with the fluorochrome Nile Red, were bright yellow while the rest of the algae cell was red (see Fig. 1-6).

In pictures 1 -6 have been shown the effect of algal cytotoxins to lipid-rich *Dunaliella* cells.

### Description of pictures 1 -6.

Picture 1: Lipid-rich *Dunaliella* cells after induced nutrient stress (40-50 % lipid content). Lipid droplets are yellow structure after staining with Nile Red (white arrows). Red structures indicate chlorophylls, proteins and carbohydrates (epifluorescence micrograph, 40X).

Picture 2: Lysing *Dunaliella* cells releasing lipid droplets (white arrows) after 1.4 h exposure to algal cytotoxins, Lipid droplets are yellow structures after staining with Nile Red. Red structures indicate chlorophylls, proteins and carbohydrates (epifluorescence micrograph, 40x).

Picture 3: *Dunaliella* cell with ruptured membrane releasing lipid droplets after 3.3h exposure to algal cytotoxins. Lipid droplets are yellow structures after staining with Nile Red (white arrows). Red structures indicate chlorophylls, proteins and carbohydrates (epifluorescence micrograph, 100x)

Picture 4: Completely lysed *Dunaliella* cell releasing lipid droplets (white arrows) after 3.3 h exposure to algae cytotoxins. Lipid droplets are yellow structures after staining with Nile Red. Red structures indicate chlorophylls, proteins and carbohydrates (epifluorescence micrograph, 100x).

Picture 5: Lysing *Dunaliella* cell releasing lipid droplets (white arrows) after 3.3h exposure to algal cytotoxins. Lipid droplets are yellow structures after staining with Nile red. Red structures indicate chlorophylls, proteins and carbohydrates (epifluorescence micrograph, 100x).

Picture 6: *Dunaliella* lipid droplets (white arrows) released to the surrounding medium after cell destruction. Lipid droplets are yellow structures after staining with Nile Red (epifluorescence micrograph, 100x).

## Claims

1. A method for recovering lipids from a lipid-producing microorganism, which comprises the following steps
- providing a biomass of a lipid-producing microorganism,
- rupturing the cell wall and/or cell membrane of said microorganism by algal cytotoxin thereby releasing lipids from the microorganism cell, and
- recovering said lipids.

2. The method according to claim 1, wherein the microorganism is a lipid-producing algae.

3. The method according to claim 1 or 2, wherein the algal cytotoxin is selected so that it ruptures the cell wall and/or cell membrane of a microalgae selected from the group consisting of genera *Phaeodactylum, Rhodomonas, Cryptomonas, Thalassiosira, Cyclotella, Haematococcus and Dunaliella, preferably of genera Phaeodactylum* and *Rhodomonas* spp.

4. The method according to any one of the preceding claims, wherein the cytotoxin producing algae is selected from the group of cyanobacteria, diatoms, dinoflagellates, prymnesiophytes and raphidophytes, preferably from the group of genera *Anabaena, Aphanizomenon, Calothrix, Cylindrospermopsis, Fisherella, Gomphosphaeria, Hapalosiphon, Microcystis, Nodularia, Nostoc, Alexandrium, Coolia, Dinophysis, Heterocapsa, Karlodinium, Karenia, Ostreopsis, Peridinium, Prorocentrum. Chrysochromulina, Phaeocystis, Prymnesium. Pseudonitzschia, Nitzschia, Heterosigma and Chattonella.*

5. The method according to any one of the preceding claims, wherein the cytotoxin producing algae is selected from the genus *Alexandrium,* most preferably from species *Alexandrium tamarense.*

6. The method according to any one of the preceding claims, wherein the lipids are recovered by extraction and/or centrifugation.

7. The method according to to any one of claims 1 to 6, wherein the lipid-producing algae cell is produced by
- harvesting the lipid-producing algae from the algae culture, and
- drying the algae cells to a water content of less than 80 weight %.

8. The method according to any one of preceding claims, wherein the microorganism/algae cells are dried by evaporation, flocculation and/or centrifugation.

9. The method according to any one of the preceding claims, wherein said algal cytotoxin is in the form of cell-free suspension of the cultivation medium of cytotoxic algae.

10. The method according to any one of the preceding claims, wherein the cytotoxin(s) is (are) incubated with the biomass 2 to 24 hours, preferably from 3 to 12 hours.

11. The method according to any one of the preceding claims, wherein the lipid-producing algae is selected from the group of Chlorophyceae (green algae), Cryptophyceae (recoiling algae), Chrysophyceae (golden brown algae), Diatomophyceae (diatoms), Dinophyceae (dinoflagellates), Euglenophyceae, Eustigmatophyceae, Pavlovophyceae, Pedinophyceae, Prasinophyceae, Prymnesiophyceae (haptophyte algae) or Raphidophyceae.

## Patentansprüche

1. Verfahren zur Rückgewinnung von Lipiden aus einem Lipid-produzierenden Mikroorganismus, welches die folgenden Schritte umfasst
- Bereitstellen einer Biomasse eines Lipid-produzierenden Mikroorganismus,
- Durchbrechen der Zellwand und/oder Zellmembran des Mikroorganismus durch Algen-Zytotoxin, wodurch Lipide aus dem Mikroorganismus freigesetzt werden, und
- Rückgewinnung der Lipide.

2. Verfahren nach Anspruch 1, wobei der Mikroorganismus eine Lipid-produzierende Alge ist.

3. Verfahren nach Anspruch 1 oder 2, wobei das Algen-Zytotoxin so ausgewählt wird, dass es die Zellwand und/oder Zellmembran einer Mikroalge, ausgewählt aus der Gruppe bestehend aus Gattungen *Phaeodactylum, Rhodomonas, Cryptomonas, Thalassiosira, Cyclotella, Haematococcus* und *Dunaliella, bevorzugt von Gattungen Phaeodactylum* und *Rhodomonas* spp, durchbricht.

4. Verfahren nach einem der vorhergehenden Ansprüche, wobei die Zytotoxin-produzierende Alge aus der Gruppe von Cyanobakterien, Diatomeen, Dinoflagellaten, Prymnesiophyten und Raphidophyten, bevorzugt aus der Gruppe von Gattungen *Anabaena, Aphanizomenon, Calothrix, Cylindrospermopsis, Fisherella, Gomphosphaeria, Hapalosiphon, Microcystis, Nodularia, Nostoc, Alexandrium, Coolia, Dinophysis, Heterocapsa, Karlodinium, Karenia, Ostreopsis, Peridinium, Prorocentrum. Chrysochromulina, Phaeocystis, Prymnesium. Pseudonitzschia, Nitzschia, Heterosigma* und *Chattonella* ausgewählt wird.

5. Verfahren nach einem der vorhergehenden Ansprüche, wobei die Zytotoxin-produzierende Alge aus der Gattung *Alexandrium,* am bevorzugtesten aus den Gattungen *Alexandrium tamarense,* ausgewählt wird.

6. Verfahren nach einem der vorhergehenden Ansprüche, wobei die Lipide durch Extraktion und/oder Zentrifugation rückgewonnen werden.

7. Verfahren nach einem der vorhergehenden Ansprüche 1 bis 6, wobei die Lipid-produzierende Algenzelle durch
- Ernten der Lipid-produzierenden Alge aus der Algenkultur und
- Trocknen der Algenzellen auf einen Wassergehalt von nicht weniger als 80 Gewichtsprozent hergestellt wird.

8. Verfahren nach einem der vorhergehenden Ansprüche, wobei die Mikroorganismus-/Algenzellen durch Verdunstung, Flockung und/oder Zentrifugation getrocknet werden.

9. Verfahren nach einem der vorhergehenden Ansprüche, wobei das Algen-Zytotoxin in Form einer zellfreien Suspension des Kultivierungsmediums von zytotoxischen Algen vorliegt.

10. Verfahren nach einem der vorhergehenden Ansprüche, wobei das Zytotoxin bzw. die Zytotoxine 2 bis 24 Stunden, bevorzugt von 3 bis 12 Stunden, mit der Biomasse inkubiert wird bzw. inkubiert werden.

11. Verfahren nach einem der vorhergehenden Ansprüche, wobei die Lipid-produzierende Alge aus der Gruppe von Chlorophyceae (grünen Algen), Cryptophyceae (recoiling-Algen), Chrysophyceae (goldbraunen Algen), Diatomophyceae (Diatomeen), Dinophyceae (Dinoflagellaten), Euglenophyceae, Eustigmatophyceae, Pavlovophyceae, Pedinophyceae, Prasinophyceae, Prymnesiophyceae (Haptophytalgen) oder Raphidophyceae ausgewählt wird.

## Revendications

1. Procédé pour la récupération des lipides à partir d'un micro-organisme producteur de lipides, qui comprend les étapes suivantes consistant à
- la fourniture d'une biomasse d'un micro-organisme producteur de lipides,
- la rupture de la paroi cellulaire et/ou de la membrane cellulaire dudit micro-organisme par cytotoxine d'algues en libérant ainsi des lipides de la cellule de micro-organisme, et
- la récupération desdits lipides.

2. Procédé selon la revendication 1, dans lequel le micro-organisme est une algue productrice de lipides.

3. Procédé selon la revendication 1 ou 2, dans lequel la cytotoxine d'algues est choisie si bien qu'elle rompt la paroi cellulaire et/ou la membrane cellulaire d'une micro-algue choisie du groupe composé par les genres *Phaeodactylum, Rhodomonas, Cryptomonas, Thalassiosira, Cyclotella, Haematococcus et Dunaliella,* préférablement les genres *Phaeodactylum* et *Rhodomonas* spp.

4. Procédé selon l'une quelconque des revendications précédentes, dans lequel les algues productrices de cytotoxine sont choisies du groupe de cyanobactéries, diatomées, dinoflagellés, prymnesiophytes et raphidophytes, de préférence du groupe des genres *Anabaena, Aphanizomenon, Calothrix, Cylindrospermopsis, Fisherella, Gomphosphaeria, Hapalosiphon, Microcystis, Nodularia, Nostoc, Alexandrium, Coolia, Dinophysis, Heterocapsa, Karlodinium, Karenia, Ostreopsis, Peridinium, Prorocentrum. Chrysochromulina, Phaeocystis, Prymnesium. Pseudonitzschia, Nitzschia, Heterosigma* et *Chattonella.*

5. Procédé selon l'une quelconque des revendications précédentes, dans lequel les algues productrices de cytotoxine sont choisies du genre *Alexandrium,* le plus préférablement des genres *Alexandrium tamarense.*

6. Procédé selon l'une quelconque des revendications précédentes, dans lequel les lipides sont récupérés par extraction et/ou centrifugation.

7. Procédé selon l'une quelconque des revendications 1 à 6, dans lequel la cellule d'algues productrices de lipides est réalisée par
- la récolte des algues productrices de lipides à partir de la culture d'algues, et
- le séchage des cellules d'algues à une teneur en eau inférieure à 80% en poids.

8. Procédé selon l'une quelconque des revendications précédentes, dans lequel les cellules de micro-organisme/d'algues sont séchées par évaporation, floculation et/ou centrifugation.

9. Procédé selon l'une quelconque des revendications précédentes, dans lequel ladite cytotoxine d'algues est dans la forme d'une suspension sans cellules du milieu de culture d'algues cytotoxiques.

10. Procédé selon l'une quelconque des revendications précédentes, dans lequel la ou les cytotoxines est/sont mise(s) en incubation avec la biomasse pendant 2 à 24 heures, de préférence de 3 à 12 heures.

11. Procédé selon l'une quelconque des revendications précédentes, dans lequel les algues productrices de lipides sont choisies du groupe de Chlorophyceae (algues vertes), Cryptophyceae (algues reculant), Chrysophyceae (algues brunes d'or), Diatomophycées (diatomées), Dinophyceae (dinoflagellés), Euglenophyceae, Eustigmatophyceae, Pavlovophyceae, Pedinophyceae, Prasinophyceae, Prymnesiophyceae (haptophyte algues) ou Raphidophyceae.
